# DEMANDE DE BREVET EUROPEEN

(11) **EP 4 000 654 A1**
(43) Date de publication de la demande: **25.05.2022**
(21) Numéro de dépôt: 21208593.0
(22) Date de dépôt: 16.11.2021
(51) Int. Cl.: A61L 2/22, A47K 7/04, A61B 90/80

(54) **APPAREIL DE DESINFECTION AVEC DISPOSITIF DE COLLECTE FLUIDIQUE**

(30) Priorité: 18.11.2020 FR 2011816
(71) Demandeur: Evercleanhand, 38420 Le Versoud (FR)
(72) Inventeur: Belle, Guillaume, 38420 LE VERSOUD (FR); Kechichian, Asbed, 38420 LE VERSOUD (FR)
(74) Mandataire: INNOV-GROUP

(57) **Abrégé**

L'invention porte sur un appareil de désinfection 1 comportant : une enceinte de désinfection (2), un dispositif d'injection (12) d'un produit désinfectant, un dispositif de collecte fluidique, lequel comporte un conduit de collecte (32) présentant une entrée (33) sur laquelle débouche une ouverture de sortie (14) de l'enceinte (2), et une sortie (34), et s'étendant entre l'entrée (33) et la sortie (34) de manière en partie courbe dans un plan principal orthogonal à la direction d'extraction (E), et comportant au moins un orifice d'écoulement (29,30) distinct de l'entrée (33) et de la sortie (34), raccordé à un circuit de récupération (10).

## Description

### DOMAINE TECHNIQUE

Le domaine de l'invention est celui des appareils de désinfection d'une solution désinfectante sous forme d'aérosol destinée à se déposer sur la surface d'un élément placé dans une enceinte de désinfection, par exemple sur les mains d'un utilisateur.

### ÉTAT DE LA TECHNIQUE ANTÉRIEURE

Dans un certain nombre de milieux, il importe que les personnes puissent se désinfecter les mains dans le but d'éviter la propagation des infections. C'est par exemple le cas, entre autres, du milieu médical pour le personnel soignant, les visiteurs et les patients, le milieu de la restauration et de l'agroalimentaire pour les opérateurs, le personnel de cuisine et les clients, ainsi que le milieu des structures d'accueil infantile et de gériatrie.

Pour cela, des appareils de désinfection existent, qui comportent une enceinte de désinfection destinée à recevoir la ou les mains à désinfecter d'un l'utilisateur, et un dispositif adapté à pulvériser ou nébuliser une solution désinfectante dans l'enceinte de désinfection.

La demande de brevet WO2018/206889A1 décrit un tel appareil de désinfection qui comporte de plus un dispositif de collecte fluidique adapté à récupérer le produit désinfectant non déposé sur l'élément à désinfecter. Dans cet exemple, le dispositif de collecte fluidique comporte un filtre de condensation situé à l'ouverture de sortie de l'enceinte de désinfection. L'aérosol non déposé sur les mains de l'utilisateur est capté par le filtre de condensation. Les microgouttelettes coalescent dans le filtre, puis le liquide s'écoule par gravité jusqu'à un réservoir.

### EXPOSÉ DE L'INVENTION

L'invention a pour objectif de remédier au moins en partie aux inconvénients de l'art antérieur, et plus particulièrement de proposer un appareil de désinfection comportant un dispositif de collecte fluidique plus performant.

Pour cela, l'objet de l'invention est un appareil de désinfection, comportant : une enceinte de désinfection, comprenant une ouverture d'entrée par laquelle un élément à désinfecter peut être introduit, et une ouverture de sortie ; un dispositif d'injection adapté à injecter dans l'enceinte de désinfection un produit désinfectant sous forme d'aérosol ; et un dispositif de collecte fluidique adapté à extraire par l'ouverture de sortie l'air présent dans l'enceinte de désinfection selon une direction d'extraction, et à collecter et canaliser par gravité dans un circuit de récupération le produit désinfectant non déposé sur l'élément à désinfecter.

Selon l'invention, le dispositif de collecte fluidique comporte un conduit de collecte présentant une entrée sur laquelle débouche l'ouverture de sortie, et une sortie, et s'étendant entre l'entrée et la sortie de manière en partie courbe dans un plan principal orthogonal à la direction d'extraction, et comportant au moins un orifice d'écoulement distinct de l'entrée et de la sortie, raccordé au circuit de récupération.

Certains aspects préférés mais non limitatifs de cet appareil de désinfection sont les suivants.

L'ouverture de sortie qui débouche sur l'entrée, et un orifice de refoulement qui débouche sur la sortie, peuvent être orientés de manière parallèle au plan principal.

Le conduit de collecte peut entourer au moins en partie l'entrée.

L'orifice d'écoulement peut être disposé à un point bas du conduit de collecte suivant un axe vertical destiné à être parallèle à l'axe de la gravité.

Le conduit de collecte peut s'étendre sensiblement en spirale depuis l'entrée jusqu'à la sortie raccordée à un orifice de refoulement d'air.

La conduite d'évacuation d'air peut comporter une buse d'extraction orientée selon une direction de refoulement parallèle à la direction d'extraction.

La sortie peut former une chambre de refoulement contenant ledit au moins un orifice d'écoulement.

La sortie peut former une chambre de refoulement contenant une rampe orientant l'air extrait en direction d'un orifice de refoulement agencé de manière parallèle au plan principal.

L'entrée du conduit de collecte peut former une chambre d'aspiration au niveau de son raccordement avec l'ouverture de sortie, le dispositif de collecte fluidique comportant un ventilateur d'extraction disposé dans la chambre d'aspiration.

L'enceinte de désinfection peut être délimitée par une paroi périphérique et par une paroi de fond, l'ouverture de sortie étant pratiquée dans la paroi de fond.

Le conduit de collecte peut être délimité par une cloison saillante sensiblement perpendiculairement de la paroi de fond.

Le conduit de collecte peut être délimité également par un couvercle sensiblement parallèle à la paroi de fond et fixé contre la cloison saillante.

La cloison saillante peut comporter une portion externe fermée sur elle-même.

L'entrée peut former une chambre d'aspiration, la cloison saillante pouvant comporter une portion interne délimitant la chambre d'aspiration.

La portion interne de la cloison saillante peut délimiter un passage d'écoulement vers ledit au moins un orifice d'écoulement.

Le circuit de récupération peut comporter, dans l'enceinte de désinfection, une portion d'écoulement en pente, dans laquelle débouche ledit au moins un orifice d'écoulement.

Le circuit de récupération peut comporter une bouche de collecte débouchant au bas de la pente de la portion d'écoulement.

La paroi périphérique peut comporter un rebord recourbé au niveau de la bouche de collecte.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
la figure 1 est une vue schématique en perspective d'un appareil de désinfection selon un mode de réalisation ;
la figure 2 est une vue de l'enceinte de désinfection de l'appareil illustré sur la fig.1 ;
la figure 3 est une vue de côté de l'enceinte de désinfection de la fig.2 et représente également le circuit de collecte de l'appareil de désinfection ;
la figure 4 est une vue éclatée des éléments visibles à la fig.2 et détaillant la construction du dispositif d'injection 12 et du dispositif d'extraction ;
la figure 5 est une vue en perspective avant de la paroi de fond de l'appareil de désinfection selon l'invention ;
la figure 6 est une vue en perspective arrière de la paroi de fond de la fig.5 ;
la figure 7 est une vue arrière de la paroi de fond de la fig.5.

### EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près, et de préférence à 5% près. Par ailleurs, les termes « compris entre ... et ... » et équivalents signifient que les bornes sont incluses, sauf mention contraire.

L'invention porte sur appareil de désinfection, par exemple par nébulisation. L'appareil de désinfection comporte ici une enceinte de désinfection adaptée à recevoir un élément à désinfecter, par exemple les mains d'un utilisateur, ainsi qu'un dispositif d'injection adapté à injecter dans l'enceinte de désinfection un aérosol d'une solution désinfectante destinée à se déposer sur l'élément introduit dans l'enceinte.

Par nébulisation, on entend la transformation en un aérosol d'un liquide initialement en phase continue. L'aérosol est une suspension ou un nuage de fines gouttelettes de liquide. La taille moyenne des gouttelettes peut être de l'ordre de quelques microns à quelques dizaines de microns, et est avantageusement inférieure à 30 µm.

L'enceinte de désinfection est délimitée par une paroi comportant une ouverture d'entrée par laquelle l'élément à désinfecter peut être introduit, et une ouverture de sortie opposée à l'ouverture d'entrée. Les ouvertures d'entrée et de sortie peuvent être opposées l'une à l'autre suivant un axe longitudinal suivant lequel s'étend l'enceinte de désinfection. L'entrée et la sortie sont des ouvertures pratiquées dans les parois délimitant l'enceinte de désinfection.

Les expressions « une ouverture d'entrée », « une ouverture de sortie », « un conduit de collecte » ne limitent pas la possibilité pour l'appareil de désinfection de posséder plusieurs de ces éléments. Ces expressions doivent donc être entendues comme « au moins une ouverture d'entrée », « au moins une ouverture de sortie », « au moins un conduit de collecte ».

Le dispositif d'injection comporte au moins un injecteur d'aérosol et peut optionnellement comporter en outre un ou plusieurs injecteurs d'air, adaptés à injecter de l'air au travers d'un orifice d'injection de la paroi suivant une direction d'injection. L'injecteur d'air et l'injecteur d'aérosol peuvent être distincts l'un de l'autre, ou confondus l'un avec l'autre.

L'appareil de désinfection comporte un dispositif de collecte fluidique, adapté à extraire l'air présent dans l'enceinte de désinfection par l'ouverture de sortie suivant une direction d'extraction, et à collecter et canaliser dans un circuit de récupération le produit désinfectant non déposé sur l'élément à désinfecter.

Comme détaillé par la suite, le dispositif de collecte fluidique comporte un conduit de collecte qui s'étend entre une entrée et une sortie de manière au moins en partie courbe dans un plan principal, lequel est sensiblement orthogonal à la direction d'extraction. Le conduit de collecte comporte au moins un orifice d'écoulement, distinct de l'entrée et de la sortie, qui est raccordé au circuit de récupération.

Un tel appareil de désinfection bénéficie d'un taux élevé de récupération de la solution désinfectante. Autrement dit, la quasi-totalité de la solution désinfectante qui est injectée dans l'enceinte de désinfection est soit déposée sur l'élément à désinfecter, soit récupérée dans le circuit de récupération, avec très peu de pertes de solution désinfectante.

L'appareil de désinfection selon l'invention garantit ainsi une importante économie en produit désinfectant en récupérant le produit désinfectant collecté dans le circuit de récupération dans le but éventuellement de le recycler. Ce recyclage peut avoir lieu au sein même de l'appareil de désinfection par un retraitement du produit récupéré, ou à l'extérieur de l'appareil suite à la collecte du produit récupéré, par exemple en bouteilles amovibles.

Cette économie de produit désinfectant est primordiale pour des applications impliquant un grand nombre de désinfections. Par exemple, dans les applications où l'ensemble des personnes pénétrant dans un bâtiment doivent obligatoirement se désinfecter les mains, la logistique relative à l'approvisionnement en produit désinfectant devient un élément critique qui peut bloquer l'accès au bâtiment en cas de manque.

Selon un mode de réalisation, l'orifice d'écoulement est disposé à un point bas du conduit de collecte. Cette notion de « point bas » est définie comme étant relative à la gravité telle qu'elle s'applique à l'appareil de désinfection dans sa position normale d'utilisation. Un point bas du conduit de collecte désigne ainsi une portion de cette conduite dans laquelle les liquides s'écoulent par gravité et peuvent s'y accumuler.

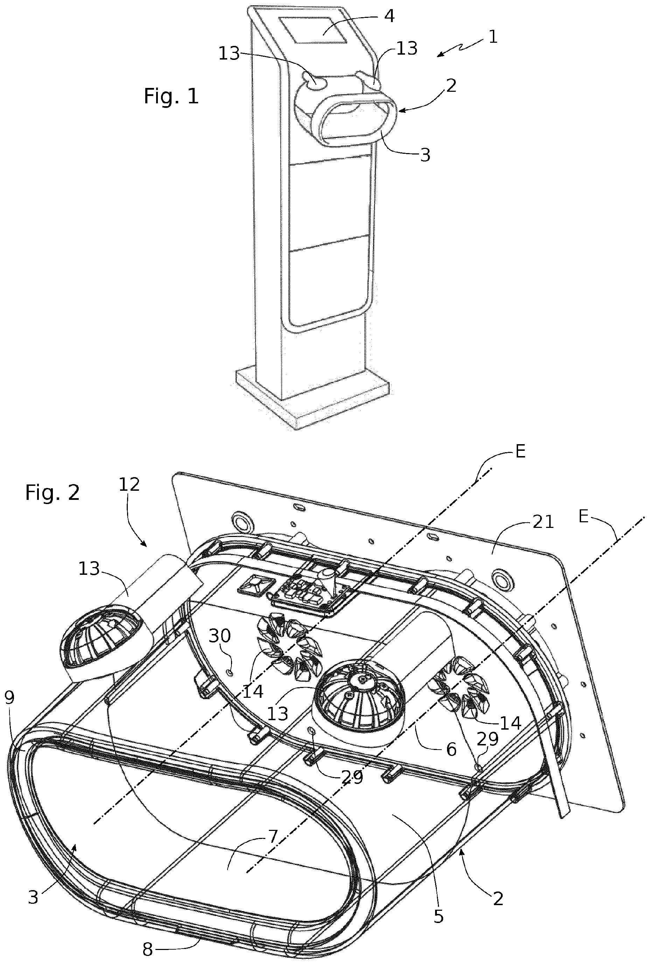

La figure 1 illustre en perspective un appareil de désinfection 1 selon un mode de réalisation. Dans le présent exemple, l'appareil de désinfection 1 est agencé sous forme d'une borne pouvant par exemple être installée à l'entrée d'un lieu ou d'un équipement qui nécessite une désinfection préalable des mains des personnes entrantes.

L'appareil de désinfection 1 est ainsi facilement accessible et comporte sur sa façade une enceinte de désinfection 2 qui est munie d'une ouverture d'entrée 3 dans laquelle l'utilisateur introduit ses deux mains dans le but de les désinfecter. L'appareil de désinfection 1 peut également comporter des interfaces 4 d'affichage et/ou de contrôle, pouvant par ailleurs être avantageusement reliées à un réseau de communication.

D'une manière générale, l'appareil de désinfection 1 comporte donc une enceinte de désinfection 2 destinée à recevoir un élément à désinfecter, un dispositif d'injection 12 d'un produit désinfectant sous forme d'aérosol dans l'enceinte de désinfection 2, et un dispositif de collecte fluidique adapté à extraire l'air présent dans l'enceinte de désinfection 2 par l'ouverture de sortie selon une direction d'extraction, et à collecter et canaliser dans un circuit de récupération le produit désinfectant non déposé sur l'élément à désinfecter.

La figure 2 est une vue en perspective de l'enceinte de désinfection 2. L'enceinte de désinfection 2 est délimitée par une paroi périphérique 5, qui est transparente dans le présent exemple, et par une paroi de fond 6 obturant la paroi périphérique 5 à l'opposé de l'ouverture d'entrée 3. La paroi périphérique 5 est sensiblement dans cet exemple un cylindre dont la base est une forme oblongue, adaptée à recevoir les deux mains d'un utilisateur. La forme de la paroi périphérique pourra être, en variante, modifiée pour s'adapter à tout autre objet à désinfecter.

La paroi périphérique 5 comporte, du côté de l'ouverture d'entrée 3, un rebord recourbé 9 délimitant l'ouverture d'entrée 3 et évitant l'écoulement par l'ouverture d'entrée 3 des fluides qui s'écouleraient le long de la paroi périphérique 5. Par rebord recourbé 9, on entend un rebord courbé vers l'intérieur de l'enceinte de désinfection 2. Il forme ainsi un conduit transversal permettant de recevoir du produit désinfectant s'écoulant par gravité vers l'ouverture de sortie 3.

La paroi périphérique 5 comporte à sa base une portion d'écoulement 7 formée ici d'une surface sensiblement plane s'étendant depuis la paroi de fond 6 jusqu'à l'ouverture d'entrée 3.

L'enceinte de désinfection 2 présente dans cet exemple une orientation inclinée vis-à-vis de l'axe de la gravité, de telle sorte que l'ouverture d'entrée 3 est agencée plus bas que la paroi de fond 6 suivant un axe vertical parallèle à l'axe de la gravité. La portion d'écoulement 7 forme ainsi un plan incliné agencé pour recueillir et drainer les liquides présents sur les faces internes de la paroi périphérique 5 et de la paroi de fond 6.

Les notions de « haut » et de « bas », ainsi que les termes « supérieur » et « inférieur », sont ici à considérer en référence au positionnement permettant l'utilisation normale de l'appareil de désinfection, tel que représenté à la fig.1.

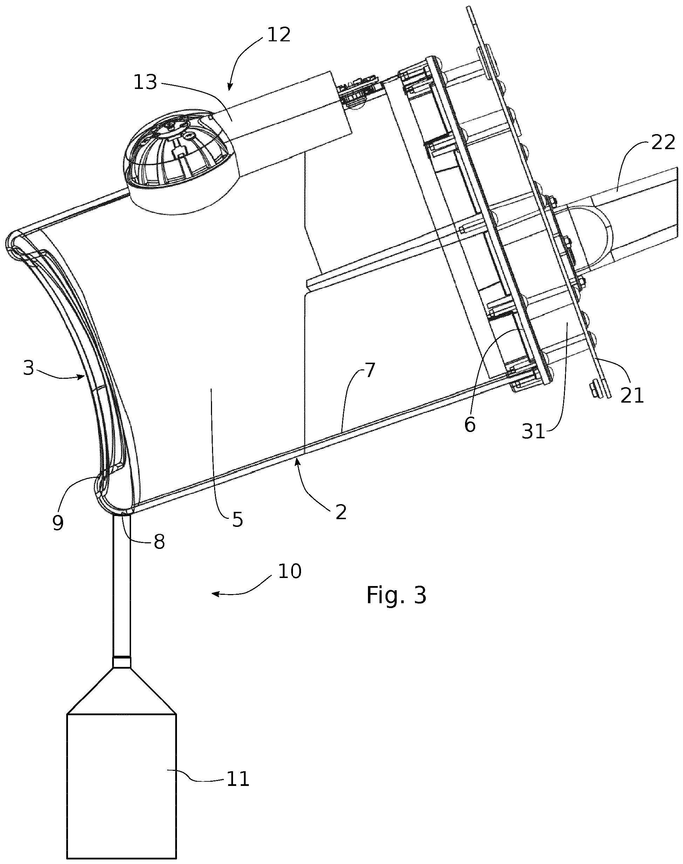

La paroi périphérique 5 comporte de plus, au niveau de la portion d'écoulement 7, du côté de l'ouverture d'entrée 3, une bouche de collecte 8 permettant la récupération des liquides s'écoulant sur la portion d'écoulement 7. Un circuit de récupération 10 est ainsi constitué notamment de la portion d'écoulement, de la bouche de collecte 8 (cf. fig.3).

La figure 3 est une vue schématique et en coupe des éléments de la fig.2, et représente plus en détail le circuit de récupération 10, lequel comporte, en plus de la portion d'écoulement 7, un réservoir de collecte 11. Le circuit de récupération 10 permet le recyclage de la solution désinfectante récupérée à la bouche de collecte 8. Dans cet exemple, le réservoir de collecte 11 peut être périodiquement vidé ou échangé dans le cadre d'un recyclage des solutions désinfectantes à l'extérieur de l'appareil de désinfection 1. En variante, le contenu du réservoir de collecte 11 peut également être retraité au sein même de l'appareil de désinfection 1 pour ensuite éventuellement être réinjecté dans l'enceinte de désinfection 2.

L'appareil de désinfection 1 comporte de plus un dispositif d'injection 12 adapté à injecter dans l'enceinte de désinfection un produit désinfectant sous forme d'aérosol. Dans le présent exemple, le dispositif d'injection 12 comporte deux têtes d'injection 13 fixées ici sur le dessus de l'enceinte de désinfection 2 et reliées à un circuit d'injection. Des têtes d'injection peuvent également être situées sur le dessous de l'enceinte de désinfection 2 et/ou sur les côtés.

De préférence, l'appareil de désinfection 1 comporte un dispositif de détection (non représenté) de la présence d'un élément à désinfecter dans l'enceinte de désinfection 2. De manière connue, le dispositif de détection peut comporter un capteur optique, par exemple une photodiode, adapté à mesurer une variation d'une caractéristique optique de l'enceinte de désinfection 2, la variation étant représentative de la présence ou non de l'élément à désinfecter. Le dispositif de détection comporte une unité de commande permettant d'activer et de désactiver le dispositif d'injection 12.

Le produit désinfectant peut être, à titre d'exemples, une solution hydroalcoolique, une solution à base d'hypochlorite de sodium ou de povidone iodée, une solution à base de chlorure d'ammonium et d'amine oxyde, de l'eau ozonée, ou toute autre solution désinfectante pouvant être nébulisée ou vaporisée.

Les gouttelettes projetées par le dispositif d'injection 12 ont une taille moyenne avantageusement inférieure à quelques dizaines de microns, par exemple 30 µm, et de préférence inférieure ou égale à 15 µm. Par exemple, la taille moyenne des gouttelettes peut être de l'ordre de 5 µm à 10 µm environ.

Tout type de nébuliseur peut être utilisé, par exemple des dispositifs de spray, des nébuliseurs à effet Venturi, ou des nébuliseurs piézoélectriques à membrane perforée vibrante au travers de laquelle un liquide s'écoule et est transformé en aérosol par la vibration de la membrane. Des exemples de nébuliseurs sont donnés notamment dans les documents FR2886174A1 ou WO2018/206899A1.

L'enceinte de désinfection 2 est de plus munie, sur la paroi de fond 6, d'ouvertures de sortie 14 associées à un dispositif d'extraction adapté à extraire, selon une direction d'extraction E, par ces ouvertures de sortie 14, l'air présent dans l'enceinte de désinfection. Les ouvertures de sortie 14 sont ici agencées en deux motifs circulaires et disposées côte à côte sur la paroi de fond 6.

L'expression « l'air présent dans l'enceinte de désinfection » désigne d'une manière générale l'ensemble formé par les gaz présents à l'intérieur de l'enceinte de désinfection, et par les éléments en suspension dans ces gaz, notamment les aérosols, cet ensemble pouvant être mis en mouvement entre l'ouverture d'entrée 3 et les ouvertures de sortie 14.

Pour utiliser l'appareil de désinfection 1, l'utilisateur insère ses mains (ou tout autre élément devant être désinfecté) dans l'enceinte de désinfection 2 par l'ouverture 3 d'entrée, ce qui entraîne la production d'un aérosol de solution désinfectante pulvérisé directement en direction des mains de l'utilisateur par le dispositif d'injection 12. Durant la pulvérisation de l'aérosol de solution désinfectante, une circulation d'air est créée en extrayant l'air de l'enceinte de désinfection 2 par les ouvertures de sortie 14.

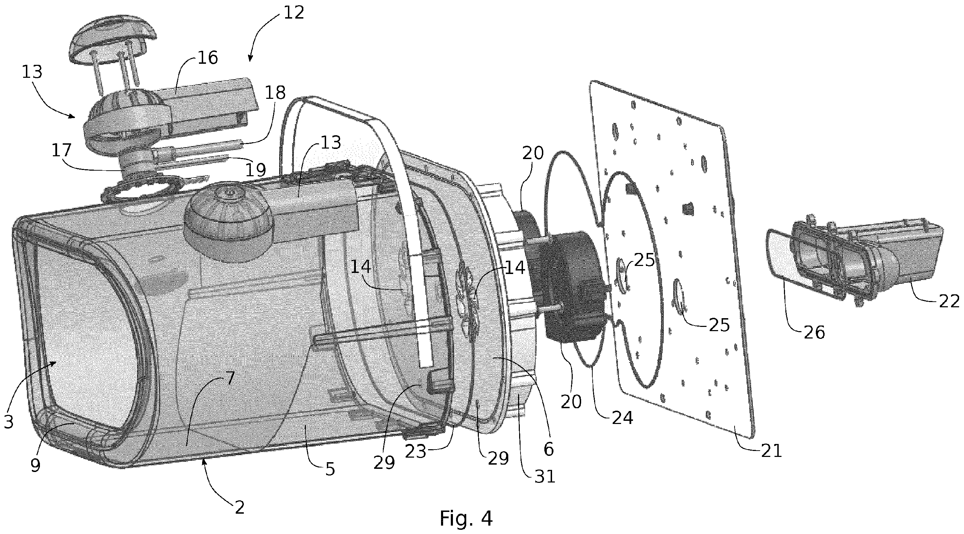

La figure 4 est une vue éclatée des éléments visibles à la fig.2 et détaillant la construction du dispositif d'injection 12 et du dispositif d'extraction.

Le dispositif d'injection 12 comporte ici deux têtes d'injection 13 dont l'une est en vue éclatée. Dans le présent exemple, chaque tête d'injection 13 comporte un capotage 16 ainsi qu'un nébuliseur 17 qui est alimenté par un conduit d'alimentation 18 en solution désinfectante sous forme liquide, et un conduit 19 de gaz sous pression permettant la nébulisation et la projection de la solution désinfectante. En variante, le dispositif d'injection peut être constitué par tout autre moyen permettant l'injection d'un aérosol au sein de l'enceinte de désinfection, comme exposé précédemment.

La vue éclatée de la fig.4 illustre également l'agencement du dispositif de collecte fluidique qui comporte, en plus du circuit de récupération, des moyens d'extraction d'air constitués ici de deux ventilateurs 20 électriques disposés entre la paroi de fond 6 et un couvercle 21, ainsi qu'une buse d'extraction 22.

La paroi de fond 6 est hermétiquement fixée contre la paroi périphérique 5 par un joint d'étanchéité 23 et les ventilateurs 20 sont eux-mêmes fixés sur la paroi de fond 6 en vis-à-vis chacun de l'un des motifs circulaires formés par les ouvertures de sortie 14. Les ventilateurs 20 peuvent ainsi extraire l'air à travers les ouvertures de sortie 14 pour les canaliser dans une chambre formée entre la paroi de fond 6 et le couvercle 21, qui sont assemblés avec un joint 24. L'air est ensuite évacué de cette chambre par deux orifices 25 du couvercle 21 débouchant dans la buse 22, cette dernière étant fixée contre le couvercle 21 avec un joint 26.

La buse 22 constitue une conduite d'évacuation d'air et est reliée à un circuit d'évacuation de l'air extrait (non représenté).

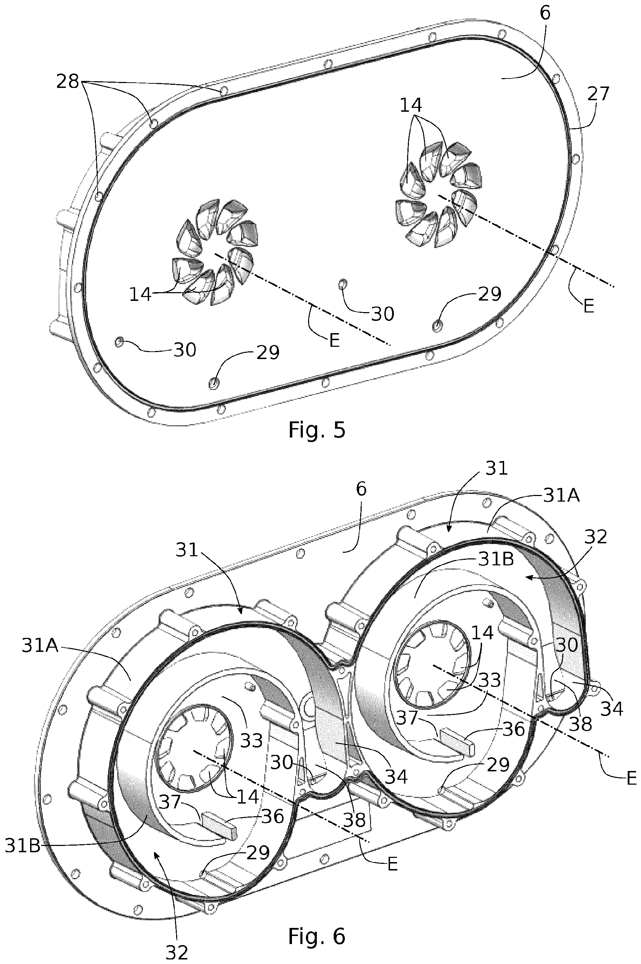

Les figures 5 et 6 illustrent en perspective la paroi de fond 6 seule, respectivement vue de trois quarts avant et de trois quarts arrière.

La face de la paroi de fond 6 qui est tournée vers l'enceinte de désinfection 2 est visible à la fig.5. Sur cette face, la paroi de fond 6 comporte une gorge 27 pour le joint 23 ainsi que des orifices 28 pour la fixation par vis de la paroi de fond 6 sur la paroi périphérique 5. La paroi de fond 6 comporte également des orifices d'écoulement 29, 30 qui permettent le retour du produit désinfectant sous forme liquide un réservoir de collecte, ici vers le réservoir 11 (cf. fig.3), par un tuyau dédié ou, comme ici, par l'intérieur de l'enceinte de désinfection 2. Les orifices d'écoulement 29, 30 sont distincts des entrée 33 et sortie 34 du conduit de collecte 32 dans le sens où ils ne constituent pas ces entrée 33 et sortie 34.

En référence à la fig.6, la paroi de fond 6 comporte sur son autre face des cloisons 31 s'étendant perpendiculairement à la surface de la paroi de fond 6 et délimitant, avec le couvercle 21, deux conduits de collecte 32. Chaque conduit de collecte 32 est associé à un ventilateur 20 et aux ouvertures de sortie 14 correspondantes. Dans cet exemple, les conduits de collecte 32 sont formés et définis par la paroi de fond 6, mais ils pourraient en être distincts, par exemple réalisés par des tuyaux éventuellement assemblés à la paroi de fond 6.

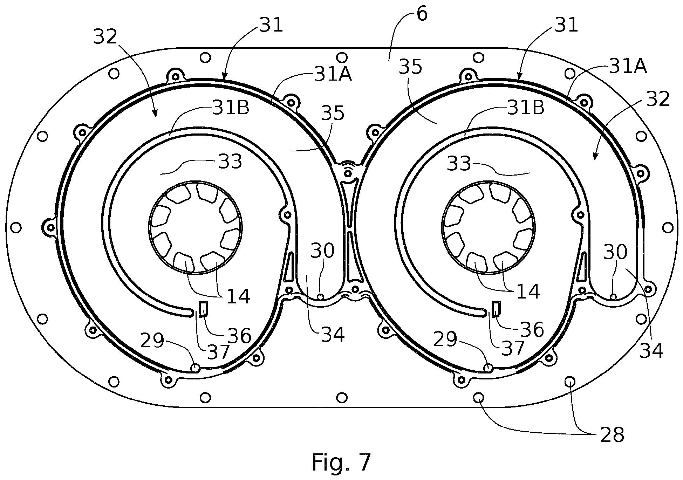

La figure 7 est une vue arrière de la paroi de fond 6 montrant la disposition des conduits de collecte 32. Chacun des conduits de collecte 32 est raccordé aux ouvertures de sortie 14, au niveau d'une chambre d'aspiration 33 formant l'entrée du conduit de collecte 32 (ou une première extrémité), et canalise l'air extrait jusqu'à une chambre de refoulement 34 formant la sortie du conduit de collecte 32 (ou une deuxième extrémité opposée à la première) dans laquelle débouche ici l'orifice de refoulement 25 correspondant du couvercle 21.

L'air extrait par les ventilateurs 20 est ainsi aspiré à travers les ouvertures de sortie 14, puis canalisé le long d'une portion courbe 35 du conduit de collecte 32, ici en spirale, jusqu'à la sortie 34 et les orifices de refoulement 25 du couvercle 21.

Ainsi, d'une manière générale, chaque conduit de collecte 32 s'étend entre l'entrée 33 (chambre d'aspiration) et la sortie 34 (chambre de refoulement) au moins en partie courbe dans un plan principal, lequel est orthogonal à la direction d'extraction E. Par orthogonal, on entend que le plan principal dans lequel s'étend le conduit de collecte 32 forme un angle de 90° environ, à plus ou moins 10° près.

De plus, l'ouverture de sortie 14 qui débouche sur l'entrée 33, et l'orifice de refoulement 25 qui débouche sur la sortie 34, sont avantageusement orientés de manière parallèle au plan principal. Ainsi, la direction d'extraction E au niveau de l'ouverture de sortie 14 ainsi que la direction de refoulement au niveau de l'orifice de refoulement sont orthogonales au plan principal.

Le conduit de collecte 32 peut comporter de plus une rampe 38 dans la chambre de refoulement 34 qui dirige l'air circulant dans le conduit de collecte 32 vers l'orifice de refoulement 25 du couvercle 21.

La portion courbe 35 donc courbe dans le plan principal. La portion 35 peut être une partie seulement du conduit de collecte 32, de sorte que celui-ci comporte également au moins une portion rectiligne, ou, comme ici, s'étendre sur toute la longueur du conduit de collecte 32. Dans cet exemple, il permet à chaque conduit de collecte 32 de décrire quasiment un tour sur lui-même entre la chambre d'aspiration 33 et la chambre de refoulement 34. En variante, le conduit de collecte 32 peut effectuer plusieurs tours selon une spirale centrée sur les ouvertures de sortie 14 correspondantes. Aussi, le conduit de collecte 32 peut entourer au moins en partie l'entrée 33 (chambre d'aspiration), améliorant ainsi la compacité de l'appareil de désinfection 1.

Les orifices d'écoulement 29, 30 sont ici percés à travers la paroi de fond 6, chacun au niveau d'un point bas du conduit de collecte 32. Un tel point bas correspond à une portion inférieure du conduit de collecte 32 dans laquelle un liquide peut s'accumuler par gravité. Dans le présent exemple, ces points bas sont au nombre de deux pour chacun des conduits de collecte 32 :
- un premier point bas correspondant à l'orifice d'écoulement 29 qui est situé au point le plus bas de l'ensemble du conduit de collecte 32, situé ici sous la chambre d'aspiration 33 ;
- un point bas correspondant à l'orifice d'écoulement 30 qui est situé dans le bas de la chambre de refoulement 34.

Par ailleurs, les cloisons 31 sont agencées pour que le conduit de collecte 32 s'étende perpendiculairement à la direction d'extraction E. Dans le présent exemple, la direction d'extraction E est perpendiculaire à la surface plane de la paroi de fond 6 et le conduit de collecte 32 s'étend ici parallèlement au plan de la paroi de fond 6. Dans cet exemple, où le conduit de collecte 32 est délimité par les cloisons 31, le conduit de collecte 32 s'étend contre la paroi de fond 6 de manière au moins en partie courbe dans le plan principal, autour du ventilateur 20 assemblé dans la chambre d'aspiration 33.

Dans le présent exemple, les cloisons 31 sont agencées, pour chaque conduit de collecte 32, en une portion externe 31A fermée sur elle-même et de forme subcirculaire, et d'une portion interne 31B s'enroulant partiellement autour du ventilateur 20 pour délimiter la chambre d'aspiration 33.

La cloison interne 31B est ici associée à son extrémité à une nervure 36, un passage 37 étant aménagé entre l'extrémité de la cloison interne 31B et la nervure 36 de sorte que tout liquide présent dans la chambre d'aspiration 33 s'écoule par le passage 37 en direction de la cloison externe 31A pour s'écouler ensuite par l'orifice d'écoulement 29.

L'air qui est extrait de l'enceinte de désinfection 2 passe ainsi à travers les ouvertures de sortie 14 selon la direction d'extraction E, puis est ensuite immédiatement canalisé selon un changement de direction à 90° environ dans le conduit de collecte 32, pour subir un trajet au moins en partie circulaire dans le plan principal, et ici en spirale autour du ventilateur 20. Ce changement de trajectoire de l'air extrait par un coude à 90° environ suivi d'un mouvement de rotation favorise le dépôt du produit désinfectant sur les parois du conduit de collecte 32, ce produit désinfectant sous forme liquide étant issu des gouttelettes formant l'aérosol de produit désinfectant présent dans l'air extrait de l'enceinte 2. Cette accumulation de liquide sur les parois du conduit de collecte 32 s'écoule ensuite par gravité le long des parois jusqu'à rejoindre l'un des orifices d'écoulement 29, 30, puis s'écoule au travers de ces orifices 29, 30 par gravité jusqu'à un réservoir, ici le réservoir 11.

En référence à nouveau aux fig. 2 et 3, le liquide désinfectant évacué par les orifices d'écoulement 29, 30 s'écoule ici le long de la portion d'écoulement 7 jusqu'à la bouche de collecte 8 et le réservoir de collecte 11 comme décrit précédemment. En variante, les orifices d'écoulement 29, 30 peuvent être directement reliés à des canalisations transférant le produit désinfectant vers le réservoir de collecte, ou vers un dispositif de recyclage.

L'air extrait de l'enceinte de désinfection 2, après son passage par le dispositif de collecte fluidique, ressort par la buse 22 débarrassé de la quasi-totalité du produit désinfectant. Dans le présent exemple, l'air extrait de l'enceinte de désinfection 2 sort par les ouvertures de sortie 14 selon la direction d'extraction E, puis parcourt un chemin en spirale perpendiculairement à la direction d'extraction E, puis est à nouveau canalisé selon une direction de refoulement par la buse 22. Les directions d'extraction et de refoulement sont avantageusement orthogonales au plan principal.

Le produit désinfectant pulvérisé par les têtes d'injection 13 est donc, pour sa quasi-totalité, soit effectivement déposé sur les mains de l'utilisateur (ou de tout autre élément à désinfecter) soit récupéré par le dispositif de collecte fluidique. Pour le produit désinfectant récupéré par le dispositif de collecte fluidique, il est soit directement récupéré par la portion d'écoulement 7, soit par le conduit de collecte 32.

Ainsi, à la différence de l'appareil de désinfection décrit dans le document WO2018/206899A1 mentionné précédemment où le dispositif de collecte est formé d'un ventilateur d'aspiration et d'un filtre de condensation, l'appareil de désinfection 1 comporte un dispositif de collecte amélioré par la présence du conduit de collecte 32 qui s'étend de manière au moins en partie courbe entre l'entrée 33 et la sortie 34, et avantageusement en spirale autour de l'ouverture 14. Ainsi, du fait de la courbure du conduit 32 et de l'écoulement de l'aérosol désinfectant, le conduit 32 présente une grande surface sur laquelle les microgouttelettes de l'aérosol viennent se déposer, de sorte que le dispositif de collecte présente une efficacité de collecte particulièrement élevée. De plus, l'efficacité d'extraction de l'aérosol hors de l'enceinte de désinfection 2 n'est pas soumise à une éventuelle saturation du dispositif de collecte, comme ça peut être le cas pour l'appareil de désinfection de l'art antérieur contenant un filtre de condensation. Enfin, la récupération de l'aérosol déposé sur la surface courbe du conduit de collecte 32 est efficace et particulièrement aisée, dans la mesure où le conduit de collecte 32 comporte au moins un orifice d'écoulement 29, 30 raccordé au circuit de récupération 10.

Des modes de réalisation particuliers viennent d'être décrits. Différentes variantes et modifications apparaîtront à l'homme du métier. Ainsi, l'élément à désinfecter peut être d'autres membres d'un utilisateur, par exemple le pied, voire être un objet introduit et maintenu par l'utilisateur dans l'enceinte de désinfection 2.

Des modes de réalisation particuliers viennent d'être décrits. Différentes variantes et modifications apparaîtront à l'homme du métier.

## Revendications

1. Appareil de désinfection (1), comportant :
∘ une enceinte de désinfection (2), comprenant une ouverture d'entrée (3) par laquelle un élément à désinfecter peut être introduit, et une ouverture de sortie (14) ;
∘ un dispositif d'injection (12), adapté à injecter dans l'enceinte de désinfection (2) un produit désinfectant sous forme d'aérosol ;
∘ un dispositif de collecte fluidique adapté à extraire par l'ouverture de sortie (14) l'air présent dans l'enceinte de désinfection (2) selon une direction d'extraction (E), et à collecter et canaliser par gravité dans un circuit de récupération (10) le produit désinfectant non déposé sur l'élément à désinfecter ;
∘ **caractérisé en ce que** le dispositif de collecte fluidique comporte un conduit de collecte (32) présentant une entrée (33) sur laquelle débouche l'ouverture de sortie (14), et une sortie (34), et s'étendant entre l'entrée (33) et la sortie (34) de manière au moins en partie courbe dans un plan principal orthogonal à la direction d'extraction (E), et comportant au moins un orifice d'écoulement (29,30) distinct de l'entrée (33) et de la sortie (34), raccordé au circuit de récupération (10).

2. Appareil de désinfection (1) selon la revendication 1, dans lequel l'ouverture de sortie (14) qui débouche sur l'entrée (33), et un orifice de refoulement (25) qui débouche sur la sortie (34), sont orientés de manière parallèle au plan principal.

3. Appareil de désinfection (1) selon la revendication 1 ou 2, dans lequel le conduit de collecte (32) entoure au moins en partie l'entrée (33).

4. Appareil de désinfection (1) selon l'une quelconque des revendications 1 à 3, dans lequel l'orifice d'écoulement (29,30) est disposé à un point bas du conduit de collecte (32) suivant un axe vertical destiné à être parallèle à l'axe de la gravité.

5. Appareil de désinfection (1) selon l'une quelconque des revendications 1 à 4, dans lequel le conduit de collecte (32) s'étend sensiblement en spirale depuis l'entrée (33) jusqu'à la sortie (34) raccordée à un orifice (25) de refoulement d'air.

6. Appareil de désinfection (1) selon la revendication 5, dans lequel l'orifice (25) de refoulement d'air est raccordé à une buse d'extraction (22) orientée selon une direction de refoulement parallèle à la direction d'extraction (E).

7. Appareil de désinfection (1) selon l'une quelconque des revendications 1 à 6, dans lequel la sortie (34) forme une chambre de refoulement contenant ledit au moins un orifice d'écoulement (30).

8. Appareil de désinfection (1) selon l'une quelconque des revendications 1 à 7, dans lequel la sortie (34) forme une chambre de refoulement contenant une rampe (38) orientant l'air extrait en direction d'un orifice (25) de refoulement d'air agencé de manière parallèle au plan principal.

9. Appareil de désinfection (1) selon l'une des revendications 1 à 8, dans lequel l'entrée (33) du conduit de collecte (32) forme une chambre d'aspiration (33) au niveau de son raccordement avec l'ouverture de sortie (14), le dispositif de collecte fluidique comportant un ventilateur d'extraction (20) disposé dans la chambre d'aspiration (33).

10. Appareil de désinfection (1) selon l'une des revendications 1 à 9, dans lequel l'enceinte de désinfection (2) est délimitée par une paroi périphérique (5) et par une paroi de fond (6), l'ouverture de sortie (14) étant pratiquée dans la paroi de fond (6).

11. Appareil de désinfection (1) selon la revendication 10, dans lequel le conduit de collecte (32) est délimité par une cloison saillante (31) sensiblement perpendiculairement de la paroi de fond (6).

12. Appareil de désinfection (1) selon la revendication 11, dans lequel le conduit de collecte (32) est délimité également par un couvercle (21) sensiblement parallèle à la paroi de fond (6) et fixé contre la cloison saillante (31).

13. Appareil de désinfection (1) selon la revendication 11 ou 12, dans lequel la cloison saillante (31) comporte une portion externe (31A) fermée sur elle-même.

14. Appareil de désinfection (1) selon l'une quelconque des revendications 11 à 13, dans lequel l'entrée (34) forme une chambre d'aspiration, la cloison saillante (31) comporte une portion interne (31B) délimitant la chambre d'aspiration (33).

15. Appareil de désinfection (1) selon la revendication 14, dans lequel la portion interne (31B) de la cloison saillante (31) délimite un passage (37) d'écoulement vers ledit au moins un orifice d'écoulement (29).
